# EUROPEAN PATENT SPECIFICATION

(11) **EP 1 218 749 B1**
(45) Date of publication and mention of the grant of the patent: **05.04.2006**
(21) Application number: 00966291.7
(22) Date of filing: 09.10.2000
(51) Int. Cl.: G01N 33/574

(54) **DETECTION OF PROSTATE CANCER MEASURING PSA/IGF-1 RATIO**
NACHWEIS VON PROSTATAKREBS DURCH BESTIMMEN DES VERHÄLTNISSES VON PSA ZU IGF-1
SYSTEME DE DETECTION DU CANCER DE LA PROSTATE PAR MESURE DU RAPPORT PSA/IGF-1

(30) Priority: 07.10.1999 GB 9923599; 27.05.2000 GB 0012869
(43) Date of publication of application: 03.07.2002
(73) Proprietor: Joken Limited, Witney, Oxon OX8 6BE (GB)
(72) Inventor: Griffiths, Keith, Castleton Cardiff (GB); Turkes, Atilla T., Cardiff CF5 2RE (GB)
(74) Representative: Drysdale, Douglas Standen
(86) International application number: PCT/GB2000/003854
(87) International publication number: WO 2001/025790

(56) References cited:
- EP-A- 0 895 085
- WO-A-99/38011
- SEITZ CHRISTIAN ET AL: "Insulin-like growth factor 1 (IGF 1), IGF 1 density of the prostate (IGFD) and IGF to PSA ratio (IGF/PSA) as new tools for prostate cancer detection." JOURNAL OF UROLOGY, vol. 161, no. 4 SUPPL., April 1999 (1999-04), page 321 XP000986922 94th Annual Meeting of the American Urological Association, Inc.;Dallas, Texas, USA; May 1-6, 1999 ISSN: 0022-5347
- DJAVAN BOB ET AL: "Insulin-like growth factor 1 (IGF-1), IGF-1 density, and IGF-1/PSA ratio for prostate cancer detection." UROLOGY, vol. 54, no. 4, October 1999 (1999-10), pages 603-606, XP000986913 ISSN: 0090-4295
- CUTTING C W M ET AL: "Serum insulin-like growth factor-1 is not a useful marker of prostate cancer." BJU INTERNATIONAL, vol. 83, no. 9, June 1999 (1999-06), pages 996-999, XP000987175 ISSN: 1464-4096
- DIAGNOSTIC SYSTEMS LABORATORIES, TECHNICAL RELEASE: INSULIN-LIKE GROWTH FACTOR-I (IGF-I) ASSAYS, [Online] 1996, pages 1 - 10 Retrieved from the Internet: <URL:http://secure.dslabs.com/Documents/tec hlit/Core%20Literature/ilgfia.pdf>

## Description

The present invention relates to a method of analysis of free prostate specific antigen (PSA) and free insulin-like growth factor-1 (IGF-1) levels to provide a more accurate differential diagnosis of prostate cancer. Advantageously the analysis of free PSA and free IGF-1 is made in a simultaneous manner, time-related changes are also monitored and a modified dual-antibody immunoassay (sandwich assay) is used.

Until recently, total prostate specific antigen (PSA) was recognised as the most sensitive and reliable index of prostatic carcinoma (Oesterling, 1993; J Urol 145: 907-923). Indeed over the last decade, particularly in the USA, it has become the major focus of prostate cancer screening programmes. However, there have been increasing concerns with respect to the fidelity and reproducibility of the measurement of and hence the value of the traditional assay procedures.

It is well documented that different commercial assays for total PSA give quantitatively different results on the same patient samples (Nakamura et al 1997. In 4^{th} International Consultation of BPH; Committee on The Detection of Prostate cancer in a patient with BPH: Ed Denis et al; SCI, Plymouth: 381-387). This creates a considerable degree of uncertainty and unreliability in the precision of the diagnosis of prostate cancer. On this basis, the committee recommended that free rather than total PSA would be a more reliable diagnostic tool. Only recently has the methodology become available for the reliable quantification of free PSA. The diagnostic value using PSA alone can be further improved by analysing the change in free PSA levels over a period of time (PSA velocity) (Oesterling et al 1993; JAMA 270: 860-864).

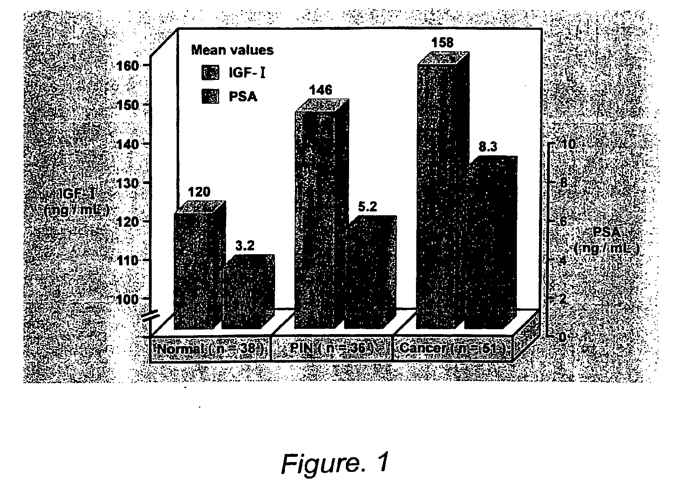

There is now considerable evidence linking the peptide growth factor, insulin-like growth factor-1 (IGF-1) to the development of prostatic hyperplasia and carcinoma. Recent work has indicated that IGF-1 levels are higher in men with prostate cancer, even when PSA is in the "normal" range. In a recent prostate cancer study in the county of Gwent, the levels of PSA have been measured in more than 3000 men between the ages of 55 and 75, and prostate cancer identified in approximately 3% of the screened group. A retrospective determination of IGF-1 in samples of serum from screened subject patients with low total PSA levels (less than 4 ng/l), those with identified pre-neoplastic lesions (PIN) and those diagnosed with cancer of the prostate, revealed a close relationship between levels of PSA and IGF-1 (see Figure 1).

Apart from the inter-assay variability, the major problem with the use of PSA as a predictive marker and test for prostate cancer is that certain cancer (about 30%) will not secrete PSA and other subjects with high PSA levels have benign prostatic hyperplasia (BPH) and not cancer. Thus, existing technology creates the opportunity for both false negative and false positive results thereby reducing both the fidelity and value of the diagnosis.

When PSA levels are greater than 20ng/ml, the probability of prostate cancer is about 90%. Levels of PSA of between 10 and 20 ng/ml could indicate cancer or benign prostatic hyperplasia (BPH). A difficulty however, lies in the assessment of patients having PSA levels, in the so-called "diagnostic grey zone", namely between 4-10 ng/ml. The levels could be normal because of old age, or could indicate a disease state and be elevated due to cancer, BPH or prostatistis. In the dual assay herein described we can differentially diagnose prostate cancer and eliminate unnecessary biopsies, particularly for those patients who exhibit PSA levels in the "diagnostic grey zone".

The analytical difficulties and the imprecision of the diagnosis are well recognised and, until recently, physician, patient and healthcare provider, alike accepted the limitations.

More recently, the use of dual markers, including PSA and IGP-1, has been advocated (see Djavan et al, 1999; Urology 54: 603-606; WO-A-99/38011). However, the differential diagnosis of prostate cancer described is based on single time point measurements of both PSA and IGP-1; concerns measurement of total PSA, (rather than free PSA); and involves different assay conditions.

Further work on the proposal to measure both IGF-1 and PSA from the same group as Djavan et al (supra) appeared in J. Urology 161/4 Suppl. 04/1999, page 321, Abstract N. 1236. Djavan et al measured total IGF-1 and concluded that the IGP-1/PSA ratio is useful for prediction of prostate cancer.

The invention provides a method for a more accurate and less ambiguous differential diagnosis of prostate cancer.

It is therefore one object of the invention to provide an assay to assist in the detection of prostate cancer in a patient, said assay comprising the step of simultaneously measuring by a sandwich immunoassay the amount of free IGF-1 and free PSA in a sample of biological fluid from said patient, or the ratio between these amounts in said sample.

Preferably several samples are collected at separate pre-determined time periods to be assayed as described above. Advantageously dual antibody techniques are used to simultaneously measure free IGP-1 and free PSA in a single sample of biological fluid. Such an assay represents a substantial qualitative and quantitative improvement on existing methodology and thereby offers the opportunity for precise differential diagnosis of prostate cancer. As both peptide hormones PSA and IGF-1 will be measured in one sample and therefore under identical assay conditions accuracy of the measurement will be provided. Fidelity of the approach is likewise considerably enriched by basing the diagnosis on free levels and on the ratio of concentration of PSA/IGF-1.

It is preferred to use a sandwich immunoassay to measure the amount of free IGF-1 and free PSA or their ratio. Such assay may further comprise the steps of :
- providing first antibodies which can specifically form immunocomplexes with free PSA and second antibodies which can form immunocomplexes with free IGF-1, these first and second antibodies being attached to a solid phase matrix; and
- contacting the solid phase matrix to the sample under conditions suitable to allow the formation of immunocomplexes;
- subsequently contacting the solid phase matrix with third antibodies which can specifically form immunocomplexes with free PSA or said first antibodies and fourth antibodies which can form immunocomplexes with free IGF-1 or second antibodies, these third and fourth antibodies being labelled and the contacting step being made under suitable conditions to allow immunocomplexes formation; and
- measuring, preferably simultaneously, the amount of labels of the third antibodies and of the fourth antibodies and/or the ratio between them.

It is also preferred that these third and fourth antibodies be labelled with distinct first and second labels.

Another aspect of the invention is a device to assist in the detection or diagnosis of prostate cancer. Such device comprises a solid phase matrix having a surface and first antibodies which can specifically form immunocomplexes with free PSA and second antibodies which can form immunocomplexes with free IGF-1 located thereon.

For example the antibodies may be chemically bound to or physically entrapped in the surface. Alternatively, the antibodies may be localised by means of electrostatic or ionic attraction. Desirably the ratio of anti-PSA antibodies: anti-IGF=1 antibodies located on said surface is in the range 1:2 to 2:1, preferably is approximately 1:1.

In a further aspect, the present invention provides a kit to assist in the detection or diagnosis of prostate cancer. The kit comprises a device as defined above, and packaged separately thereto, third antibodies which can specifically form immunocomplexes with free PSA or said first antibodies and fourth antibodies which can specifically form immunocomplexes with free IGF-1 or second antibodies, said third and fourth antibodies being labelled with first and second labels, respectively. Advantageously, the kit may further comprise means to detect and measure the amount of label(s) retained on said apparatus following its exposure to said labelled antibodies.

The above generally describes the present invention which can be better understood with reference to the following example.

### Brief Description of the Figure.

Figure 1 shows mean levels of PSA and IGF-1 for patients with low levels of PSA and the correlation with prostate cancer.

### Example

The principle of the conventional two-site (sandwich) immunoassay which is preferred requires two antibodies, one of which is linked to a solid-phase matrix in order to provide a means of separation, and the other is "labelled" to allow the end point determination. Both antibodies recognise the analyte to be measured and form an antibody-analyte-antibody immunocomplex. The amount of complex formed is directly related to the concentration of analyte present in the serum sample.

Two different antibodies raised against PSA and IGF-1 are linked to the solid phase matrix and two corresponding labelled antibodies will be used to measure both analytes, PSA and IGF-1, simultaneously in a single sample of biological fluid. Assay conditions can be optimised to provide the analytical sensitivity required for the detection of free PSA and free IGF-1.

The sample may be diluted with suitable like BSA (Bovine serum albumine), PBS (Phosphate Buffer Saline), or BSA (Bovine gamma globuline) before being contacted with the solid phase matrix. The sample is then allowed to incubate for a period of time, like a few hours, to allow for the formation of immunocomplexes and the solid phase matrix is then washed, for example with PBS, to remove non-immunocomplexed material. Then the solid matrix can be contacted with labelled antibodies and then incubated and washed before the amount of labels being measured.

The mono- and/or poly-clonal antibodies raised against PSA and IGF-1, can be purified either using salt fractionation, or Protein-A or affinity purification procedures and together will be attached to a solid phase matrix. The solid phase matrix can be prepared by linking antibodies to plastic test tubes, beads, microtitre plates/strips, microcrystalline cellulose, glass or magnetic particles, or membranes used in semiquantitative assays. Particularly preferred support is tubes or microtitre plates.

Suitable commercially available anti-PSA antibodies include Nos. 7820-0004 and 7820-0350 of Biogenesis Ltd, Poole, Dorset, UK; MAB4082 of Chemicon International Inc, Temecula, CA, USA; SC-7638 and SC-7816 of Santa Cruz Biotechnology Inc, Santa Cruz, CA, USA; and MAS 343cf of Harlan Sera-Lab Ltd, Loughborough, Leicestershire, UK.

Suitable commercially available anti-IGF antibodies include Nos. 5345-0329 and 5345-0209 of Biogenesis Ltd, Poole, Dorset, UK; GF006 of Chemicon International Inc, Temecula, CA, USA; SC-7144 and SC-1422 of Santa Cruz Biotechnology Inc, Santa Cruz, CA, USA; and MAS 974p of Harlan Sera-Lab Ltd, Loughborough, Leicestershire, UK.

The second pair of antibodies recognising the different antigenic determinants of free PSA and free IGF-1 can be used with labels. Labelled antibodies can be prepared by conjugating with enzymes (horseradish peroxidase or alkaline phosphate), or a fluorogenic agent (fluorocein), or a chemiluminescent agent (luminol or an acridinium derivative). It is proposed to use rare earth chelates, like europium and samarium, in the preparation of the labelled antibodies. On the basis of their different absorption/emission characteristics, they can be used at the same time in an assay system: europium for labelled IGF-1 antibody and samarium for PSA, or vice versa. However, other pairs of labels like erythin/iron can also be effective in giving different emissions easy to differentiate.

The principle of underlying the invention, therefore, is that antibodies against free PSA and IGF-1 on the same solid matrix, will form antibody-analyte complexes with free PSA and free IGF-1 in the same sample of serum and labelled PSA and IGF-1 will bind to their respective analytes. This completes the antibody-analyte-antibody sandwich. The end point analysis will be undertaken on an instrument capable of determining time-lapse, or time-resolve fluorescence, like a spectrophotometer.

## Claims

1. An assay to assist in the detection of prostate cancer in a patient, said assay comprising the step of simultaneously measuring by a sandwich immunoassay the amount of free IGF-1 and free PSA in a sample of biological fluid from said patient, or the ratio between these amounts in said sample.

2. The assay claimed in Claim 1, wherein more than one sample from the same patient taken at separate and pre-determined time periods is submitted to the assay.

3. The assay of Claim 2, wherein said assay further comprises the steps of :
- providing first antibodies which, can specifically form immunocomplexes with free PSA and second antibodies which can form immunocomplexes with free IGF-1, said first and second antibodies being attached to a solid phase matrix; and
- contacting the solid phase matrix of said sample under conditions suitable to allow the formation of immunocomplexes;
- subsequently contacting said solid phase matrix with third antibodies which can specifically form immunocomplexes with free PSA or said first antibodies and fourth antibodies which can form immunocomplexes with free IGF-1 or second antibodies, said third and fourth antibodies being labelled, said contacting step being made under suitable conditions to allow immunocomplex formation; and
- measuring the amount of labels of the third antibodies and of the fourth antibodies and/or the ratio between them.

4. The assay of Claim 3, wherein said third and fourth antibodies are labelled with distinct first and second labels.

5. A device to assist in the detection of prostate cancer, said device comprising a solid phase matrix having a surface and first antibodies which can specifically form immunocomplexes with free PSA and second antibodies which can form immunocomplexes with free IGF-1.

6. The device as claimed in Claim 5, wherein the ratio of first and second antibodies provided on the surface is ranging from 1:2 to 2:1.

7. The device of Claim 6, wherein said ratio is approximately 1:1.

8. The device of any one of Claims 5 to 7, wherein said solid phase matriX is a plastic test tube, beads, microtitre plates/strips, microcrystalline cellulose, glass or magnetic particles or a membrane used in a semi-quantitative assay.

9. A kit to assist in the detection of prostate cancer, said kit comprising a device as described in any one of Claims 5 to 7 and, packaged separately, third antibodies which can specifically form immunocomplexes with free PSA or said first antibodies and fourth antibodies which can form immunocomplexes with free IGF-1, or second antibodies, said third and fourth antibodies being labelled with first and second labels, respectively.

10. The kit of Claim 9, wherein said first and second labels are distinct from each other and are colour labels which under suitable conditions generate distinct absorption/emission characteristics so that the amount of first and second label can be measured simultaneously.

11. The kit of Claims 9 or 10, wherein said first and second labels are distinct from each other and are chosen in the group consisting of enzymes, fluorogenic agents and chemiluminescent agents.

12. The kit of Claim 9 or 10, wherein said first and second labels are distinct from each other and comprise rare earth chelates.

13. The kit of Claim 12, wherein said first and second labels are distinct from each other and comprise either europium or samarium chelates.

14. The kit of Claim 9 or 10, wherein said first and second labels are distinct from each other and comprise either erythin or iron.

15. The kit of any of Claims 9 to 12, wherein said kit further comprises means to detect the amount of labels retained on the device following its exposure to said labelled antibodies.

## Revendications

1. Dosage conçu pour faciliter le dépistage d'un cancer de la prostate chez un patient, ledit dosage comprenant l'étape qui consiste à mesurer simultanément, par un immunodosage de type sandwich, les taux d'IGF-1 libre et de PSA libre dans un échantillon de liquide biologique obtenu chez ledit patient, ou le rapport de ces taux dans ledit échantillon.

2. Dosage selon la revendication, qui est effectué sur plus d'un échantillon obtenus chez un même patient à des intervalles de temps distincts prédéterminés.

3. Dosage selon la revendication 2, comprenant également les étapes qui consistent à:
- utiliser des premiers anticorps qui peuvent spécifiquement former des immunocomplexes avec le PSA libre et des deuxièmes anticorps qui peuvent former des immunocomplexes avec l'IGF-1 libre, lesdits premiers et deuxièmes anticorps étant fixés sur une matrice en phase solide; et
- mettre en contact la matrice en phase solide avec ledit échantillon dans des conditions appropriées à la formation d'immunocomplexes ;
- mettre ensuite en contact ladite matrice en phase solide avec des troisièmes anticorps qui peuvent spécifiquement former des immunocomplexes avec le PSA libre ou avec lesdits premiers anticorps, et avec des quatrièmes anticorps qui peuvent former des immunocomplexes avec l'IGF-1 libre ou avec lesdits deuxièmes anticorps, lesdits troisièmes et quatrième anticorps étant marqués et ladite étape de mise en contact étant réalisée dans des conditions appropriées à la formation d'immunocomplexes ; et
- mesurer les taux de marquage des troisièmes anticorps et des quatrièmes anticorps, et/ou le rapport entre ces taux.

4. Dosage selon la revendication 3, dans lequel lesdits troisièmes et quatrièmes anticorps sont marqués au moyen d'un premier et d'un second traceurs distincts.

5. Dispositif conçu pour faciliter le dépistage du cancer de la prostate, ledit dispositif comprenant une matrice en phase solide qui présente une surface, des premiers anticorps qui peuvent spécifiquement former des immunocomplexes avec le PSA libre et des deuxièmes anticorps qui peuvent former des immunocomplexes avec l'IGF-1 libre.

6. Dispositif selon la revendication 5, dans lequel le rapport entre les taux des premiers et des deuxièmes anticorps fixés sur la surface est compris entre 1 : 2 et 2 : 1.

7. Dispositif selon la revendication 6, dans lequel ledit rapport est de l'ordre de 1 : 1.

8. Dispositif selon l'une quelconque des revendications 5 à 7, dans lequel ladite matrice en phase solide correspond à un tube à essai en plastique, des billes, des plaques/bandes de microtitrage, la cellulose microcristalline, des particules de ou verre magnétiques ou une membrane utilisés dans un dosage semi-quantitatif.

9. Trousse conçue pour faciliter le dépistage du cancer de la prostate, ladite trousse comprenant un dispositif tel que décrit à l'une quelconque des revendications 5 à 7 et, emballés séparément, des troisièmes anticorps qui peuvent spécifiquement former des immunocomplexes avec le PSA libre ou avec lesdits premiers anticorps et des quatrièmes anticorps qui peuvent former des immunocomplexes avec l'IGF-1 libre ou avec les deuxièmes anticorps, lesdits troisièmes et quatrièmes anticorps étant marqués par un premier et second traceurs, respectivement.

10. Trousse selon la revendication 9, dans laquelle lesdits premier et second traceurs sont distincts l'un de l'autre et correspondent à des traceurs colorés qui, dans des conditions appropriées, génèrent des caractéristiques d'absorption/d'émission distinctes permettant une mesure simultanée des taux du premier et du second traceurs.

11. Trousse selon la revendication 9 ou 10, dans laquelle lesdits premier et second traceurs sont distincts l'un de l'autre et sont sélectionnés parmi le groupe consistant en des enzymes, des agents fluorogènes et des agents chimioluminescents.

12. Trousse selon la revendication 9 ou 10, dans laquelle lesdits premier et second traceurs sont distincts l'un de l'autre et comprennent des chélates de terres rares.

13. Trousse selon la revendication 12, dans laquelle lesdits premier et second traceurs sont distincts l'un de l'autre et comprennent des chélates d'europium ou de samarium.

14. Trousse selon la revendication 9 ou 10, dans laquelle lesdits premier et second traceurs sont distincts l'un de l'autre et comprennent de l'érythrine ou du fer.

15. Trousse selon l'une quelconque des revendications 9 à 12, ladite trousse comprenant également un moyen de détecter la quantité de traceurs retenus sur le dispositif après son exposition auxdits anticorps marqués.

## Patentansprüche

1. Assay zum Erleichtern des Erfassens von Prostatakrebs bei einem Patienten, wobei der Assay den Schritt des gleichzeitigen Messens, durch einen Sandwichimmunassay, der Menge an freiem IGF-1 und an freiem PSA in einer Probe von biologischem fluid eines des Patienten oder des Verhältnisses zwischen diesen Mengen in der Probe umfasst.

2. Assay nach Anspruch 1, wobei mehr als eine von demselben Patienten zu getrennten und vorbestimmten Zeiten abgenommene Probe dem Assay unterworfen wird.

3. Assay nach Anspruch 2, wobei der Assay des Weiteren folgende Schritte umfasst:
- Bereitstellen erster Antikörper, die mit freiem PSA spezifisch Immunkomplexe bilden können, und zweiter Antikörper, die mit freiem IGF- 1 Immunkomplexe bilden können,wobei die ersten und zweiten Antikörper an eine Festphasenmatrix gebunden sind; und
- Kontaktieren der Festphasenmatrix der Probe unter Bedingungen, die geeignet sind, die Bildung von Immunkomplexen zu erlauben;
- darauffolgendes Kontaktieren der Festphasenmatrix mit dritten Antikörpern, die mit freiem PSA oder den ersten Antikörpern spezifisch Immunkomplexe bilden können, und vierten Antikörpern, die mit freiem IFG-1 oder zweiten Antikörpern Immunkomplexe bilden können, wobei die dritten und vierten Antikörper gelabelt sind, wobei der Kontaktierschritt unter geeigneten Bedingungen durchgeführt wird, um die Immunkomplexbildung zu erlauben; und
- Messen der Menge an Labeln der dritten Antikörper und der vierten Antikörper und/oder des Verhältnisses dazwischen.

4. Assay nach Anspruch 3, wobei die dritten und vierten Antikörper mit getrennten ersten und zweiten Labeln gelabelt werden.

5. Gerät zum Erleichtern der Erfassung von Prostatakrebs, wobei das Gerät eine Festphasenmatrix mit einer Oberfläche und erste Antikörper, die mit freiem PSA spezifisch Immunkomplexe bilden können, und zweite Antikörper, die mit freiem IGF-1 Immunkomplexe bilden können, umfasst

6. Gerät nach Anspruch 5, wobei das Verhältnis erster und zweiter Antikörper, die auf der Oberfläche bereitgestellt sind, im Bereich von 1:2 bis 2: 1 liegt

7. Gerät nach Anspruch 6, wobei das Verhältnis ca. 1:1 ist.

8. Gerät nach einem der Ansprüche 5 bis 7, wobei die Festphasenmatrix ein Kunststoffreagenzglas, Perlen, Mikrotiterplatten/-streifen, mikrokristalline Zellulose, Teilchen von oder Glas magnetischen Teilchen, und eine Membran, die in einem halbquantitativen Assay verwendet wird, ist

9. Kit zum Unterstützen der Erfassung von Prostatakrebs, wobei der Kit ein Gerät, wie in einem der Ansprüche 5 bis 7 beschrieben, und getrennt verpackt dritte Antikörper, die mit freiem PSA oder den ersten Antikörpern spezifisch Immunkomplexe bilden können, und vierte Antikörper, die mit freiem IGF-1 oder zweiten Antikörpern Immunkomplexe bilden können, umfasst, wobei die dritten und vierten Antikörper jeweils mit einem ersten und zweiten Label gelabelt sind.

10. Kit nach Anspruch 9, wobei die ersten und zweiten Label voneinander verschieden und Farblabel sind, die unter geeigneten Bedingungen verschiedene Absorptions-/Emissionscharakteristiken bilden, so dass die Menge an erstem und zweitem Label gleichzeitig gemessen werden kann.

11. Kit nach Anspruch 9 oder 10, wobei die ersten und zweiten Label voneinander verschieden sind und unter der Gruppe ausgewählt werden bestehend aus Enzymen, fluorogenen Mitteln und chemilumineszierenden Mitteln.

12. Kit nach Anspruch 9 oder 10, wobei die ersten und zweiten Label voneinander verschieden sind und Seltenerdechelate umfassen.

13. Kit nach Anspruch 12, wobei die ersten und zweiten Label voneinander verschieden sind und entweder Europium- oder Samariumchelate umfassen.

14. Kit nach Anspruch 9 oder 10, wobei die ersten und zweiten Label voneinander verschieden sind und entweder Erythin oder Eisen umfassen.

15. Kit nach einem der Ansprüche 9 bis 12, wobei der Kit des Weiteren Mittel zum Erfassen der Menge an Labeln umfasst, die auf dem Gerät auf dessen Aussetzen den gelabelten Antikörper gegenüber hin zurückgehalten werden.
